# EUROPÄISCHE PATENTSCHRIFT

(11) **EP 3 473 181 B1**
(45) Veröffentlichungstag und Bekanntmachung des Hinweises auf die Patenterteilung: **19.02.2020**
(21) Anmeldenummer: 17197523.8
(22) Anmeldetag: 20.10.2017
(51) Int. Cl.: A61B 6/00, G01R 33/54, G06F 3/16, G10L 15/22

(54) **VERFAHREN ZUM BETRIEB EINER MEDIZINISCHEN BILDAUFNAHMEEINRICHTUNG, BILDAUFNAHMEEINRICHTUNG, COMPUTERPROGRAMM UND ELEKTRONISCH LESBARER DATENTRÄGER**
IMAGE RECORDING DEVICE, METHOD FOR OPERATING A MEDICAL IMAGE RECORDING DEVICE, COMPUTER PROGRAM AND ELECTRONICALLY READABLE DATA CARRIER
PROCÉDÉ DE FONCTIONNEMENT D'UN DISPOSITIF D'ACQUISITION D'IMAGES MÉDICALES, DISPOSITIF D'ACQUISITION D'IMAGES, PROGRAMME INFORMATIQUE ET SUPPORT D'INFORMATIONS LISIBLE ÉLECTRONIQUEMENT

(43) Veröffentlichungstag der Anmeldung: 24.04.2019
(73) Patentinhaber: Siemens Healthcare GmbH, 91052 Erlangen (DE)
(72) Erfinder: Lerch, Daniel, 91365 Weilersbach (DE); Lerch, Kathrin, 91365 Weilersbach (DE)

(56) Entgegenhaltungen:
- DE-A1-102014 226 467
- US-A1- 2015 237 222
- US-A1- 2016 104 293

## Beschreibung

Die Erfindung betrifft ein Verfahren zum Betrieb einer medizinischen Bildaufnahmeeinrichtung im Rahmen einer Untersuchung eines mit einem Patientenpositionierungsmittel positionierten Patienten. Daneben betrifft die Erfindung eine medizinische Bildaufnahmeeinrichtung, ein Computerprogramm und einen elektronisch lesbaren Datenträger.

Medizinische Bildaufnahmeeinrichtungen, mit denen Untersuchungen von Patienten durchgeführt werden, werden in der modernen Medizin immer häufiger eingesetzt. Mit Hilfe moderner bildgebender Verfahren werden zwei- oder dreidimensionale Bilddaten erzeugt, die zur Visualisierung eines abgebildeten Untersuchungsobjekts und darüber hinaus auch für weitere Anwendungen genutzt werden können. Gerade bei komplexeren Bildgebungsverfahren, beispielsweise bei der Magnetresonanzbildgebung und der Computertomographie, steigt häufig die Untersuchungsdauer an, so dass ein Patient, mittels eines Patientenpositionierungsmittels, beispielsweise einer Patientenliege, positioniert, eine längere Zeit in einem Bildgebungsbereich der Bildaufnahmeeinrichtung verbringen muss.

Trotz Fortschritten durch neue Bedienkonzepte sind Patienten nach wie vor über gewisse Zeitstrecken während der Untersuchung in medizinischen Bildaufnahmeeinrichtungen sich selbst überlassen. Dies gilt insbesondere für die Zeit der eigentlichen Untersuchung/Bildaufnahme, um so Bedienpersonen vor entstehender Strahlung bzw. vor entstehendem Lärm zu schützen. Patienten können im Untersuchungsraum auch sich selbst überlassen sein, wenn bei der Bildaufnahmeeinrichtung, beispielsweise einer Magnetresonanzeinrichtung, die Vorbereitung der Untersuchung (Protokolleinstellungen und dergleichen) außerhalb des Untersuchungsraums stattfindet.

Eine bildgebende Untersuchung stellt für einen Patienten ohnehin bereits eine Situation großer Unsicherheit dar. Diese Untersicherheit wird weiter gesteigert, wenn die Bedienperson ihn nach der Positionierung in der Bildaufnahmeeinrichtung verlässt, um die Untersuchungseinstellungen vorzunehmen und das Ende der Untersuchung, beispielsweise in einem Kontrollraum, abzuwarten. Dabei ist zwar grundsätzlich vorgesehen, dass die Bedienperson im näheren Einwirkungs- und vor allem Hörbereich des Patienten verbleibt, was aber nicht in allen medizinischen Einrichtungen, beispielsweise Kliniken, möglich ist. Beispielsweise dann, wenn nur eine geringe Belegschaft vorhanden ist, können Ablenkungen durch andere Patienten, Kollegen, Rückfragen von der Anmeldung, Notfälle und/oder Ärzte (Radiologen) dazu führen, dass Äußerungen bzw. sonstige Geräusche des alleine in der Bildaufnahmeeinrichtung wartenden Patienten überhört werden.

Beispielsweise kann es vorkommen, dass ein Patient Fragen stellt, beispielsweise bezüglich der Untersuchung, des einzunehmenden Zielzustandes und dergleichen. Insbesondere bei sehr jungen oder sehr alten Patienten kann das Spektrum der Rückfragen hierbei breitgefächert sein. Bleiben derartige Fragen des Patienten unbeantwortet, kann sich beispielsweise die Unsicherheit des Patienten erhöhen, was zu höherer Atem-und/oder Herzfrequenz führen kann, folglich zu Bilddaten potentiell schlechterer Qualität bei entsprechend bewegten Untersuchungsobjekten. Eine weitere Folge unbeantworteter Fragen kann sein, dass der Patient, beispielsweise sich allein gelassen fühlend oder in dem Glauben, die Untersuchung sei bereits zu Ende, zu früh aufsteht und somit die sorgfältig für die Untersuchung vorbereitete Positionierung verlässt.

Kritische Situationen können auch auftreten, wenn ein Patient gesundheitliche Probleme aufweist, beispielsweise unter einer Kontrastmittelunverträglichkeit oder Platzangst leidet. Wird eine entsprechende verbale Äußerung von der Bedienperson nicht wahrgenommen, kann dies zu gesundheitlichen Problemen des Patienten führen. Hierfür wurde vorgeschlagen, dem Patienten eine Notbedieneinrichtung, beispielsweise einen Notstoppknopf, bereitzustellen, wobei jedoch dann, wenn der Patient kognitiv bzw. körperlich nicht in der Lage ist, das Notbedienelement zu betätigen, ein Überhören des Patienten ebenso ungünstige Folgen haben kann.

Kinder als Patienten stellen ein weiteres Problem dar. Die Erfahrung zeigt, dass pädiatrische Patienten aufgrund ihres großen Bewegungsdrangs meist nicht ruhig liegen bleiben, wenn sie beispielsweise auf einer Patientenliege als Patientenpositionierungsmittel platziert wurden. Dies führt dazu, dass die Bedienperson oder eine andere Begleitperson im Untersuchungsraum verbleiben müssen, was beispielsweise aufgrund von Strahlen- und/oder Lärmexposition unerwünscht sein kann.

Neben sinkender Qualität von aufgenommenen Bilddaten und potentiellen gesundheitlichen Problemen führen die vorangehend genannten Situationen auch dazu, dass die erlebte Qualität der Untersuchung für den Patienten negativ beeinflusst wird, insbesondere was das Offenbleiben von Fragen, Angst oder gar Schmerzen angeht. Diese erlebte Qualität ist es häufig, die am Ende die Bewertung der Untersuchung durch den Patienten prägt, nachdem dieser nicht über die Möglichkeiten verfügt, zu bewerten, wie gut oder wie schlecht die Diagnose selbst war. Diese Bewertung, ob direkt über Bewertungsportale im Internet oder aber indirekt durch Mund-zu-Mund-Propaganda oder Rücksprache mit dem zuweisenden Arzt, wird zu einem wirtschaftlichen einflussreichen Faktor, der definieren kann, wie viele öffentlichen Mittel einem Gesundheitsversorger zugewiesen werden oder aber wie hoch der Patientenzustrom ist.

Um diese Probleme zu mindern, wurde vorgeschlagen, zumindest bei Großgeräten als medizinische Bildaufnahmeeinrichtungen ein Paar von Mikrofon und Lautsprecher vorzusehen, welches auch als Interkom bezeichnet werden kann. Über ein derartiges Interkom kann der Patient mit der Bedienperson Kontakt aufnehmen und umgekehrt. Nach dem das Interkom auch in beide Richtungen stumm geschaltet werden kann, wird die Gefahr eines Überhörens des Patienten wiederum erhöht.

Ein weiteres bekanntes Hilfsmittel ist das bereits erwähnte Notbedienelement, beispielsweise als Notstoppknopf, welches der Patient während der Untersuchung in der Hand hält und mit welchem er beim Auftreten körperlicher oder psychischer Probleme die Untersuchung abbrechen kann. Ein derartiges Notbedienelement wirkt jedoch in dem Sinne binär, dass bei Überschreiten der Toleranzschwelle des Patienten nach Betätigung des Notbedienelements die Untersuchung abgebrochen wird. Ein gutes Zureden, beispielsweise durch eine Bedienperson, um die Situation zu bewältigen, ist dann nicht mehr möglich.

Auch bekannt, z.B. aus der US2015/0237222 A1, sind Bildaufnahmeeinrichtungen, bei denen vom Operateur gesprochene Befehle von Spracherkennungseinheiten analysiert und von der medizinischen Bildaufnahmeeinrichtung ausgeführt werden. Der Patient wird dabei nicht überwacht, so dass keine Informationen vom Patienten aufgenommen und analysiert werden.

Der Erfindung liegt daher die Aufgabe zugrunde, eine verbesserte Möglichkeit zur Interaktion mit einem Patienten anzugeben, um Untersuchungsabbrüche und/oder schlechte Bildqualitäten verbessert zu vermeiden.

Zur Lösung dieser Aufgabe ist bei einem Verfahren der eingangs genannten Art erfindungsgemäß vorgesehen, dass die Bildeinnahmeeinrichtung ein Sprachinteraktionssystem mit wenigstens einem Geräusche des Patienten aufnehmenden Mikrofon und wenigstens einem Lautsprecher für den Patienten aufweist, wobei
- von dem Patienten stammende Geräusche mittels des Mikrofons aufgenommen werden und von einer Spracherkennungseinheit des Sprachinteraktionssystems zur Ermittlung einer den Zustand des Patienten und/oder den Inhalt gesprochener Worte beschreibenden Patienteninformation ausgewertet werden,
- in Abhängigkeit von der Patienteninformation eine Sprachausgabe über den Lautsprecher an den Patienten erfolgt.

Erfindungsgemäß wird mithin vorgeschlagen, dem Patienten einen virtuellen Gesprächspartner bereit zu stellen, wie dieser aus verschiedenen Alltagsanwendungen, beispielsweise von Mobiltelefonen oder Multimediasystemen, bereits bekannt ist, wobei ein derartiger virtueller Gesprächspartner als Sprachinteraktionssystem auf die speziellen Anforderungen bei medizinischen Bildaufnahmeeinrichtungen, insbesondere Großgeräten, abzustellen ist. Das Sprachinteraktionssystem kann dabei helfen, negative Folgen vom Abbruch der Untersuchung bis hin zur Verletzung des Patienten abzumildern oder zu verhindern, bzw. zumindest die vom Patienten erlebte Untersuchungsqualität zu verbessern. Der virtuelle Gesprächspartner erkennt und analysiert Geräusche des Patienten, insbesondere die Sprache des Patienten, und kann so mit ihm sprachlich interagieren und mithin zu einem hilfreichen Assistenten einer Bedienperson werden. Ein derartiges, Spracherkennung und kommunizierende Assistenz-KI kombinierendes Sprachinteraktionssystem kann beispielsweise als "LIA" (Learning and Interacting Assistant) bezeichnet werden.

Dabei können bevorzugt bereits bei der medizinischen Bildaufnahmeeinrichtung verbaute Mikrofone und/oder Lautsprecher auch für das Sprachinteraktionssystem genutzt werden, um den Patienten zu "hören". Beispielsweise können für ein Interkom vorgesehene Mikrofone und Lautsprecher auch zusätzlich für das Sprachinteraktionssystem eingesetzt werden.

Dabei ist, wie bereits angedeutet, die vorliegende Erfindung besonders vorteilhaft auf medizinische Großgeräte anwendbar, beispielsweise Magnetresonanzeinrichtungen, Computertomographieeinrichtungen, SPECT-Einrichtungen und PET-Einrichtungen als Bildaufnahmeeinrichtungen. Als Patientpositionierungsmittel wird bei solchen Bildaufnahmeeinrichtungen, aber auch bei anderen medizinischen Bildaufnahmeeinrichtungen, häufig eine Patientenliege eingesetzt.

Insgesamt erlaubt es ein derartiges Patienteninteraktionssystem, das einen virtuellen Gesprächspartner bereit stellt, dafür zu sorgen, dass sich Patienten in den Zeiten, in denen sie alleine im Untersuchungsraum sind, besser versorgt fühlen. Die Zeit, bis sich eine Bedienperson wieder persönlich um sie kümmern kann, kann dem Wohlbefinden des Patienten zuträglich überbrückt werden. Insbesondere sind, worauf im Folgenden noch näher eingegangen werden wird, Fälle denkbar, in denen das Bedürfnis des Patienten nach Information oder Kommunikation vollständig von dem Sprachinteraktionssystem erfüllt werden kann, während sich die Bedienperson der Steuerung der Bildaufnahmeeinrichtung oder anderen Aufgaben widmen kann.

Dabei ist bereits bei dieser Stelle anzumerken, dass insbesondere bei Kindern als Patienten, die Akzeptanz virtueller Gesprächspartner besonders hoch ist. Mithin kann der Einsatz der vorliegenden Erfindung bei Kindern nicht nur zu einer deutlichen Verbesserung des Untersuchungserlebnisses führen (weniger Angst, mehr Spaß), sondern auch die Patientenkooperation verbessern, welche gerade bei pädiatrischen Patienten essentiell ist, um Bilddaten frei von Bewegungsartefakten zu halten und dergleichen.

In zweckmäßiger Weiterbildung der Erfindung kann vorgesehen sein, dass bei der Auswertung der Geräusche als Patienteninformation eine Anfrageklasse ermittelt wird, wobei den Anfrageklassen eine die Sprachausgabe definierende Ausgabeinformation zugeordnet ist oder wird. Nachdem Patienten Anfragen auf unterschiedliche Art und Weise stellen können, aber dennoch den Erhalt derselben Information beabsichtigen bzw. durch sonstige Lautäußerungen auf unterschiedliche Art und Weise einen Zustand kundtun, ist es zweckmäßig, im Rahmen der Spracherkennung diese unterschiedlichen Arten der Kommunikation, die aber mit einer vergleichbaren Reaktion beantwortet werden kann, zu Anfrageklassen zusammen zu fassen. Dabei kann selbstverständlich auch eine Anfrageklasse für nicht interpretierbare Geräusche vorgesehen werden, auf die selbstverständlich auch eine entsprechende Reaktion des virtuellen Gesprächspartners erfolgen kann, beispielsweise eine Nachfrage, ob geholfen werden kann oder ob eine Aussage wiederholt werden kann.

Zur Spracherkennung können dabei im Stand der Technik bereits grundsätzlich bekannte Spracherkennungsalgorithmen eingesetzt werden, welche insbesondere auch auf künstliche Intelligenz basieren können. Mit solchen Spracherkennungsalgorithmen, welche von der Spracherkennungseinheit genutzt werden können, können beispielsweise vom Patienten gesprochene Worte interpretiert werden und beispielsweise auf Schlüsselworte oder Schlüsselausdrücke hin untersucht werden, um eine zugehörige Anfrageklasse aufzufinden.

Beispielsweise kann vorgesehen sein, dass wenigstens eine eine beantwortbare Patientenfrage des Patienten beschreibende Anfrageklasse verwendet wird. Beispielsweise können hier bestimmte Schlüsselworte oder Schlüsselausdrücke als Ergebnis eines Spracherkennungsalgorithmus analysiert werden, um das konkrete Informationsbedürfnis des Patienten identifizieren zu können. In einem konkreten Beispiel können als Patientenfragen entsprechenden Anfrageklassen Anfragen zu einem Untersuchungsablauf verwendet werden, insbesondere zu einem Untersuchungsbeginn und/oder einer Untersuchungsdauer und/oder zu einem aktuellen oder folgenden Untersuchungsschritt. Typische Fragen von Patienten umfassen beispielsweise "Wann beginnt die Untersuchung?", "Wie lange dauert die Untersuchung noch?" oder "Was passiert als nächstes?".

Eine besonders vorteilhafte Weiterbildung sieht in diesem Kontext vor, dass bei einer eine in einer Steuereinrichtung der Bildaufnahmeeinrichtung vorliegenden, der Patientenfrage zuordenbaren Antwortinformation die Ausgabeinformation unter Aufnahme der Antwortinformation gebildet wird. Die Antworten auf viele Patientenfragen liegen innerhalb der medizinischen Bildaufnahmeeinrichtung bereits elektronisch vor, so dass die entsprechenden Patientenfragen automatisiert durch das Sprachinteraktionssystem beantwortet werden können, indem die in der Bildaufnahmeeinrichtung vorliegende Antwortinformation in die Ausgabeinformation integriert wird. So ist es möglich, ohne Eingreifen durch die Bedienperson das Bedürfnis des Patienten nach Information oder Kommunikation zu befriedigen.

Eine Weiterbildung der Erfindung sieht vor, dass bei einer einen Kontaktwunsch mit einem menschlichen Bedienpersonal ausdrückenden Patientenfrage eine Anwesenheitsanfrage an eine Kommunikationseinrichtung wenigstens einer Bedienperson gesendet und ein, insbesondere den Namen der kontaktierten Bedienperson enthaltender, Wartehinweis als Sprachausgabe an den Patienten ausgegeben wird. Beispielsweise kann auf einfache, mit der Spracherkennungseinheit detektierte Anfragen wie "Hallo?" oder "Wo sind Sie?" das Sprachinteraktionssystem mit einer Sprachausgabe wie "Einen Moment - ich stelle eine Verbindung zu [Name der Bedienperson] her" antworten und automatisch die Bedienperson auf die Anfrage des Patienten aufmerksam machen, insbesondere durch Senden einer Anwesenheitsanfrage an wenigstens eine der wenigstens einen Bedienperson zugeordnete Kommunikationseinrichtung. Bei der Kommunikationseinrichtung kann es sich insbesondere auch um ein Mobiltelefon und/oder DECT-Telefon handeln. Die Bedienperson sieht sodann den Bedarf des Patienten und kann sich diesem wieder zuwenden.

In einer weiteren, bevorzugten Ausgestaltung der vorliegenden Erfindung kann vorgesehen sein, dass wenigstens eine ein Unwohlsein des Patienten beschreibende Anfrageklasse verwendet wird, wobei als Ausgabeinformation ein den Patienten beruhigender Ansprachetext mit wohlwollender Stimmlage ausgegeben wird und eine Notfallnachricht an wenigstens eine wenigstens einer Bedienperson zugeordnete Kommunikationseinrichtung ausgegeben wird. Durch die Geräusche, insbesondere Kommunikation, des Patienten kann auf verschiedene Grade von Unwohlsein gefolgert werden. Beispielsweise kann für unterschiedliche Grade von Unwohlsein jeweils eine Anfrageklasse vorgesehen werden. Hinweise auf ein Unwohlsein geben beispielsweise Sätze des Patienten wie "Wann kann ich endlich hier raus?", "Mein Arm tut weh!" oder "Ich habe Angst!". Bei Feststellen einer solchen, ein Unwohlsein des patientenbeschreibenden Anfrageklasse kann der virtuelle Gesprächspartner mit wohlwollender Stimme beruhigend auf den Patienten einreden und derweil, insbesondere als stummen Alarm, eine Notfallnachricht an die wenigstens eine Kommunikationseinrichtung der wenigstens einen Bedienperson senden. Dabei können den verschiedenen Anfrageklassen, die verschiedene Arten von Unwohlsein beschreiben können, selbstverständlich auch unterschiedliche Maßnehmen zugeordnet sein, so dass beispielsweise bei Klaustrophobie ein medizinisch-technischer Radiologie-Assistent (MTRA) herbeigerufen werden kann, bei Anzeichen einer vorher unbekannten Kontrastmittelunverträglichkeit gegebenenfalls auch unmittelbar ein Notfallmediziner als Bedienperson.

Eine besonders vorteilhafte Ausgestaltung ist dann gegeben, wenn, insbesondere bei einem Kind als Patienten, ein den Patienten beschäftigender, spielerischer Dialog geführt wird, insbesondere durch einen Algorithmus der künstlichen Intelligenz. Während sich derartige Algorithmen der künstlichen Intelligenz für virtuelle Gesprächspartner im allgemeinen bereits zur Beschäftigung von Patienten eignen, lässt sich bei Kindern mit einem solchen virtuellen Gesprächspartner zusätzlich erreichen, dass auf die Begleitung durch eine Bedienperson oder durch eine Begleitperson, die versucht, das Kind während der Untersuchung ruhig zu halten, möglicherweise verzichtet werden kann. Gerade Kinder haben einen besonderen Spaß daran, mit virtuellen Gesprächspartnern zu interagieren. Eine solche spielerische Interaktion kann dazu genutzt werden, das Kind in ein Gespräch zu verwickeln, das es in der Vorbereitung der Untersuchung ablenkt und es dann, wenn die Untersuchung beginnt, spielerisch dazu bringt, den Atem anzuhalten oder einfach nur ruhig liegen zu bleiben.

Mithin ist es auch bei Kindern besonders bevorzugt, jedoch auch bei sonstigen Patienten vorteilhaft einsetzbar, wenn wenigstens eine Sprachausgabe, insbesondere ein Dialogabschnitt des Dialogs, dem Herstellen eines Zielzustandes des Patienten in Abhängigkeit von einem Untersuchungsprotokoll dient. Während, allgemein gesprochen, Sprachausgaben des Sprachinteraktionssystem vorteilhaft genutzt werden können, um Patienten Hinweise hinsichtlich des optimalen Zielzustandes während der Untersuchung zu geben, beispielsweise also das Sprachinteraktionssystem einen Patienten dazu anleiten könnte, sich selbständig zu positionieren, bietet sich gerade bei Kindern die Möglichkeit, den pädiatrischen Patienten spielerisch zu verbesserter Patientenkooperation zu bewegen, wobei insbesondere auch eine Art Training durchgeführt werden kann, bis der Patient den entsprechenden Zielzustand, beispielsweise für eine vorbestimmte Zeitdauer beibehalten kann. Insbesondere kann in diesem Kontext vorgesehen sein, dass der Zielzustand ein Atemanhalten und/oder ein Stillhalten für eine vorbestimmte Zeitspanne betrifft, wobei das Herstellen des Zielzustandes als ein Triggersignal für eine die Untersuchung steuernde Steuereinrichtung der Bildaufnahmeeinrichtung verwendet wird.

Bei einem Kind kann die Sprachausgabe, insbesondere im Rahmen des erwähnten Dialogs, beispielsweise genutzt werden, den Patienten spielerisch dazu zu bringen, den Atem anzuhalten oder einfach nur ruhig zu liegen. Beispielsweise könnte ein Sprachausgabe der Art "Ich wette, Du kannst den Atem keine 10 Sekunden anhalten. Atme mal ein, ich zähle dann mit." erfolgen. Dies kann gegebenenfalls als eine Art "Atemcoaching" wiederholt werden, bis schließlich die eigentliche Bildaufnahme der Untersuchung abhängig von dem Gesprächsverlauf des Sprachinteraktionssystems mit dem Patienten gesteuert wird. Dann löst nicht ein zeitlicher Trigger ein akustisches, automatisiertes Atemanhaltekommando aus, sondern das Sprachinteraktionssystem erzeugt selbständig ein Triggersignal, sobald erkannt wird, dass der Patient wirklich den Atem anhält (bzw. stillhält). Bei einem Kind könnte eine Sprachausgabe zum Halten einer Zielposition beispielsweise "So wie ich Dich kenne, kannst Du sicher keine 10 Sekunden ruhig liegen bleiben." enthalten.

In einer konkreten Ausgestaltung kann vorgesehen sein, dass die Herstellung des Zielzustandes durch die Auswertung von Sensordaten wenigstens eines den Patienten überwachenden Patientensensors überprüft wird. Derartige, oft ohnehin bei medizinischen Bildaufnahmeeinrichtungen vorgesehene Patientensensoren können beispielsweise Kameras und/oder Abstandsensoren umfassen. Die Sensordaten entsprechender Sensoren ermöglichen es, festzustellen, ob der entsprechende Zielzustand tatsächlich hergestellt ist.

Mit besonderem Vorteil kann vorgesehen sein, dass bei Feststellung einer Veränderung des Patientenzustands vom Zielzustand die Untersuchung abgebrochen wird und/oder wenigstens eine Bedienperson benachrichtigt wird und/oder mittels einer Sprachausgabe der Patient zum Wiederherstellen des Zielzustands angeregt wird. Dann kann beispielsweise vorgesehen sein, dass bei Wiederherstellen des Zielzustands ein Neustart-Triggersignal erfolgt, um zumindest einen Schritt der Bildaufnahme zu wiederholen.

Bewegt sich beispielsweise ein Patient, nachdem eine Bedienperson das Zimmer verlassen hat und wird dies von einem Patientensensor der Bildaufnahmeeinrichtung erkannt, kann das Sprachinteraktionssystem entsprechend reagieren. Beispielsweise kann eine wohlmeinende Sprachausgabe vorgesehen werden, die den Patienten anregt, noch eine Weile ruhig liegen zu bleiben, bis die Untersuchung beginnt. Sind Kinder beispielsweise beim Atemanhalten / Stillhalten nicht kooperativ, insbesondere im Rahmen eines solchen "Coachings" bzw. "Trainings", kann als Sprachausgabe ein freundlicher Tadel der Art "Ha! Ich hab's gesehen - Du hast es keine 10 Sekunden geschafft! Siehst Du, Du kannst es doch nicht!" erfolgen. Antwortet das Kind nun beispielsweise mit "Kann ich wohl!", kann das Sprachinteraktionssystem mit "Glaub ich nicht, das musst Du mir beweisen!" fortsetzen.

Auch bei erwachsenen Patienten ist eine entsprechende Ausgestaltung jedoch äußerst nützlich. So kann auch ein erwachsener Patient, der sich nicht mehr in einer idealen Position, also nicht mehr im Zielzustand befindet, über sprachliche Interaktion angeleitet werden, den Zielzustand wieder einzunehmen, beispielsweise der Art "Kannst Du bitte noch einmal ganz gerade nach oben für mich schauen? ... Danke!" Damit können beispielsweise Positionierungsfehler und/oder sonstiges Fehlverhalten des Patienten, nachdem eine Bedienperson den Untersuchungsraum verlassen hat, nicht nur erkannt, sondern sogar selbständig ohne Interaktion mit der Bedienperson behoben werden.

Neben dem bereits erwähnten, insbesondere im Rahmen des Dialogs durchführbaren "Coaching", bei dem entsprechende Aufforderungen mehrmals wiederholt werden, bis der Zielzustand für die vorbestimmte Zeitspanne gehalten wird, ehe dann, sobald eine gewisse Stabilität erreicht ist, auch tatsächlich die Bildaufnahme durchgeführt wird, ist es auch zweckmäßig, gegebenenfalls ein Wartezeitintervall nach Einnahme des Zielzustands fortzusehen, um einen Puffer bereit zu stellen, in dem überprüft wird, ob der Zielzustand auch tatsächlich gehalten werden wird. Entsprechende Anforderungen durch die Sprachausgabe können um das Wartezeitintervall zusätzlich zu der geplanten Messzeit ergänzt sein.

Viele heute gängige Spracherkennungsalgorithmen und somit unter Nutzung derselben verwendete Spracherkennungseinheiten sind auf diese aktuell nutzende Personen einzustellen, um eine verlässliche Spracherkennung zu ermöglichen. Eine Ausgestaltung der vorliegenden Erfindung sieht in diesem Kontext vor, dass zur Kalibrierung der Spracherkennungseinheit auf einen Patienten vorbestimmte, von dem Patienten gesprochene Worte aufgenommen und zur Kalibrierung analysiert werden. Konkret kann hierbei vorgesehen sein, dass bei Einflechtung der vorbestimmten Worte in einen Dialog des Patienten mit einer Bedienperson und/oder Anforderung der vorbestimmten Worte durch die Bedienperson mittels einer Eingabevorrichtung ein das Sprechen der vorbestimmten Worte anzeigendes Kalibrierungssignal nach einer entsprechenden Betätigung eines Bedienelements durch die Bedienperson erzeugt und an die Spracherkennungseinheit geschickt wird. Zur Initialisierung des virtuellen Gesprächspartners kann beispielweise das üblicherweise durchgeführte Gespräch einer Bedienperson mit dem Patienten während der Positionierung und dem Aufklären des Patienten genutzt werden. In diesem Gespräch können entweder für den Patienten nicht erkennbar konkrete Schlüsselworte untergebracht werden, mit deren Hilfe die Spracherkennungseinheit lernt, die Stimme des Patienten zu erkennen. Alternativ kann der Patient auch direkt gebeten werden, die vorbestimmten Worte, insbesondere als Initialisierungssätze, zu sprechen. Mittels eines Bedienelements kann die Bedienperson dem Sprachinteraktionssystem mitteilen, wann die entsprechenden Worte gesprochen werden, so dass eine gezielte Auswertung und Einstellung auf den jeweiligen Patienten erfolgen kann. Beispielsweise kann hierzu ein Tablet-PC, wie er bereits zum Einsatz bei solchen Vorgängen vorgeschlagen wurde, verwendet werden.

In diesem Kontext sieht eine vorteilhafte Weiterbildung vor, dass die Eingabevorrichtung, insbesondere der erwähnte Tablet-PC, selbst wenigstens ein Mikrofon aufweist, das zusätzlich zu dem Mikrofon des Sprachinteraktionssystems die vorbestimmten Worte aufnimmt, wobei die jeweiligen Aufnahmen gemeinsam zur Kalibrierung ausgewertet werden. Auf diese Weise können mithin in die Eingabevorrichtung, insbesondere ein Tablet, integrierte Mikrofone benutzt werden, um die Aufnahmequalität bei der Kalibrierung (Initialisierung) zu verbessern.

Eine bevorzugte Ausgestaltung der Erfindung sieht vor, dass bei der Auswertung der Geräusche zusätzlich Bilddaten eines auf den Patienten gerichteten optischen Patientensensors berücksichtigt werden, insbesondere bei Verwendung einer ein Unwohlsein des Patienten beschreibenden Anfrageklasse. Zusätzlich zur eigentlichen Spracherkennung bzw. zur Erkennung von akustischen Triggern, also Geräuschen, die neben der Sprache Unwohlsein offenbaren, können insbesondere auch optische Patientensensoren dafür verwendet werden, um festzustellen, ob ein Eingreifen seitens des Sprachinteraktionssystems sinnvoll ist bzw. ein stiller Alarm ausgelöst werden muss, wie beschrieben wurde. Diese Ausgestaltung wird bevorzugt zusätzlich zu der Überwachung von Zielzuständen, wie sie beschrieben wurde, anhand von Patientensensoren eingesetzt.

Dabei sei noch angemerkt, dass die Patientensensoren im allgemeinen, also insbesondere optische Sensoren (Kameras) und/oder Abstandssensoren, neben einer gemeinsamen Auswertung mit über das wenigstens eine Mikrofon aufgenommenen Geräuschen auch unabhängig betrachtet werden können, beispielsweise, um Momente, in denen ein Patient tatsächlich unbewegt ist bzw. den Atem anhält, für die Bildaufnahme abzupassen. In diesem Fall kann das Triggern von Bildaufnahmen komplett durch die Bildaufnahmeeinrichtung mit dem Sprachinteraktionssystem erfolgen, insbesondere bei auch durch das Sprachinteraktionssystem erzeugbaren Triggersignalen, wie oben erläutert. Auf diese Weise werden letztlich ähnliche Ansätze verfolgt wie beim EKG-Triggern und/oder Atemtriggern mit entsprechenden Sensoriken bereits grundsätzlich bekannt ist. Auch in diesen Fällen kann die Bildaufnahmeeinrichtung letztlich selbst entscheiden, wann Bilddaten mit möglichst geringen Bewegungsartefakten aufgenommen werden können. An die Stelle eines EKG-Signals bzw. Atemsignals kann dann die Patientensensorik treten, insbesondere gepaart mit einem Atem-und/oder Stillhaltecoaching durch den virtuellen Gesprächspartner.

Vorzugsweise kann eine Anzeigevorrichtung zur Anzeige eines mit Sprachausgaben über den Lautsprecher synchronisierten Avatar des Sprachinteraktionssystems verwendet werden. Insbesondere in Kulturkreisen, in denen ein virtueller Gesprächspartner in einem Untersuchungsraum eher steril und/oder unmenschlich wirkt, kann dieser Effekt abgemildert werden, wenn der virtuellen Stimme des virtuellen Gesprächspartners auch ein virtueller Avatar zur Darstellung beigestellt wird. Dabei kann beispielsweise konkret vorgesehen sein, dass der Avatar auf das Innere einer Patientenaufnahme und/oder Gantry projiziert wird und/oder auf wenigstens einem Display als Anzeigevorrichtung dargestellt wird. Eine besonders vorteilhaft nutzbare Ausgestaltung sind beispielsweise auch bereits für Magnetresonanzeinrichtungen und dergleichen vorgeschlagene Auskleidungen der Patientenaufnahme/Gantry mit OLEDs als Anzeigevorrichtung.

Eine weitere mögliche Ausgestaltung der Anzeigevorrichtung ist eine Holographieeinrichtung. Diese ermöglicht es, den Avatar als ein Hologramm wiederzugeben, zu dass eine futuristisch anmutende Darstellung des virtuellen Gesprächspartners steht, die insbesondere mit einer größeren Zahl an Freiheitsgraden im Raum vorgenommen werden kann, woraus auch eine größere Realitätsnähe resultieren kann.

In einer besonders bevorzugten Ausgestaltung der Erfindung kann vorgesehen sein, dass als Anzeigevorrichtung eine Augmented-Reality-Brille oder eine Virtual-Reality-Brille verwendet wird, die der Patient trägt. Derartige, die Wahrnehmung erweiternde oder ersetzende Hilfsmittel sind im Stand der Technik bereits grundsätzlich bekannt, beispielsweise im Fall einer Augmented-Reality-Brille als die "HoloLens" der Firma Microsoft. Eine derartige Brille (AR oder VR) ermöglich eine besonders realitätsnahe und flexible Wiedergabe des Avatars. Insbesondere eröffnet die Verwendung einer derartigen Anzeigevorrichtung jedoch auch weitere, vorteilhafte Optionen.

So sieht eine Weiterbildung vor, dass die Augmented-Reality-Brille oder Virtual-Reality-Brille zur Darstellung einer beruhigend auf den Patienten wirkenden Alternativumgebung verwendet wird, wobei insbesondere in einem Untersuchungsraum, in dem die Untersuchung stattfindet, vorhandene Gegenstände, insbesondere durch eine brillenseitige Sensorik, detektiert und durch Objekte der Alternativumgebung ersetzt werden. Dies ermöglicht es mithin, zumindest einen Teil der sterilen, möglicherweise unfreundlich wirkenden Umgebung des Untersuchungsraums durch eine Alternativumgebung mit beruhigenden Eigenschaften zu ersetzen, in die auch der Avatar eingebunden ist. Beispielsweise können als Alternativumgebung Strandlandschaften und/oder Berglandschaften und/oder Waldlandschaften verwendet werden, ggf. nach Wunsch des Patienten. Durch diese Landschaften können beispielsweise weiße Krankenhauswände und/oder Zubehörschränke überdeckt werden.

Nachdem die Überdeckung mit virtuellen Objekten, die durch die Augmented-Reality-Brille oder die Virtual-Reality-Brille ermöglicht wird, insbesondere im letzteren Fall zur vollständigen Ersetzung der Umgebung, dazu führen kann, dass der Patient, beispielsweise bei Bewegungen im Untersuchungsraum, Kollisionsobjekte übersehen kann und/oder die Wechselwirkung mit Gegenständen, beispielsweise der Patientenliege als Patientenpositionierungsmittel, problematisch werden kann, ist zweckmäßigerweise vorgesehen, solche Gegenstände zu lokalisieren und innerhalb der Alternativumgebung durch passende virtuelle Objekte zur Kollisionsvermeidung und/oder Interaktion zu ersetzen. So kann beispielsweise vorgesehen sein, mittels Sensorik der Brille selbst Kollisionsobjekte zu detektieren und/oder Positionsinformationen einer Patientenliege und/oder sonstiger Komponenten der Bildaufnahmeeinrichtung von dieser zu erhalten. Mögliche Kollisionsobjekte werden durch auch in der Alternativumgebung zu umgehenden Objekte ersetzt, bei einer Waldlandschaft beispielsweise durch Bäume. Das Patientenpositionierungsmittel kann beispielsweise als ein einladendes Bett innerhalb der Alternativumgebung dargestellt werden. Der Avatar kann dem entsprechenden virtuellen Objekt beigestellt werden, beispielsweise mit der Bitte, Platz zu nehmen.

Durch die Verwendung einer Augmented-Reality-Brille oder einer Virtual-Reality-Brille kann dem Patienten mithin die Angst vor der Untersuchung weiter genommen werden, wobei der Avatar des Sprachinteraktionssystems insbesondere als Führer innerhalb einer Alternativumgebung wirken kann. Die Bereitschaft des Patienten, die Untersuchung trotz eigener Ängste durchzuführen, wird weiter erhöht.

Neben dem Verfahren betrifft die Erfindung auch eine Bildaufnahmeeinrichtung, aufweisend ein zur Durchführung des erfindungsgemäßen Verfahrens ausgebildetes Sprachinteraktionssystem. Neben den üblichen Komponenten weist die Bildaufnahmeeinrichtung mithin auch ein Sprachinteraktionssystem mit wenigstens einem Geräusche des Patienten aufnehmenden Mikrophon und wenigstens einem Lautsprecher zu dem Patienten auf. Die Steuereinrichtung des Sprachinteraktionssystems ist bevorzugt mit einer Steuereinrichtung der Bildaufnahmeeinrichtung, die auch dem Bildaufnahmebetrieb steuert, zu einer gemeinsamen Steuereinrichtung integriert. Eine derartige Steuereinrichtung des Sprachinteraktionssystems kann neben der Spracherkennungseinheit auch eine Analyseeinheit aufweisen, die in Abhängigkeit von der Patienteninformation die Sprachausgabe über den Lautsprecher wählt und steuert. Als Patientenpositionierungsmittel kann bevorzugt eine Patientenliege vorgesehen sein. Die Bildaufnahmeeinrichtung kann als eine Magnetresonanzeinrichtung und/oder eine Computertomographieeinrichtung und/oder eine SPECT-Einrichtung und/oder eine PET-Einrichtung ausgebildet sein. Bevorzugt umfasst die Bildaufnahmeeinrichtung und/oder das Sprachinteraktionssystem auch wenigstens einen Patientensensor, eingesetzt wie oben beschrieben. Sämtliche Ausführungen bezüglich des erfindungsgemäßen Verfahrens lassen sich analog auf die erfindungsgemäße Bildaufnahmeeinrichtung übertragen, mit welcher mithin ebenso die bereits genannten Vorteile erhalten werden können.

Ein erfindungsgemäßes Computerprogramm ist beispielsweise direkt in einen Speicher einer Steuereinrichtung einer Bildaufnahmeeinrichtung ladbar und weist Programmmittel auf, um die Schritte eines hierin beschriebenen Verfahrens auszuführen, wenn das Computerprogramm in der Steuereinrichtung der Bildaufnahmeeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinrichtung einer Bildaufnahmeeinrichtung ein hierin beschriebenes Verfahren durchführen. Bei dem elektronisch lesbaren Datenträger handelt es sich bevorzugt um einen nichttransienten Datenträger, beispielsweise eine CD-ROM.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: einen Ablaufplan eines Coachings im erfindungsgemäßen Verfahren, und
- Fig. 3: eine erfindungsgemäße Bildaufnahmeeinrichtung.

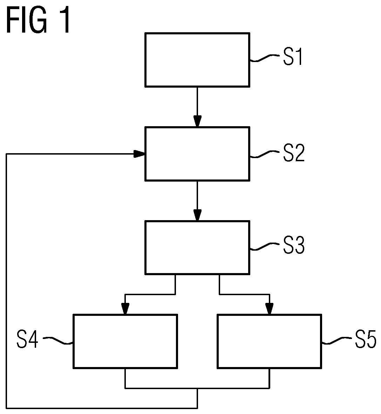

Fig. 1 zeigt einen Ablaufplan eines Ausführungsbeispiels des erfindungsgemäßen Verfahrens. Mit dem hier dargestellten Ausführungsbeispiel wird ein Sprachinteraktionssystem für eine medizinische Bildaufnahmeeinrichtung realisiert, beispielsweise für eine Computertomographieeinrichtung und/oder eine Magnetresonanzeinrichtung. Das Sprachinteraktionssystem weist wenigstens ein Mikrofon in einem Untersuchungsraum auf, mit dem Geräusche eines dort zu untersuchenden Patienten aufgenommen werden können. Ferner umfasst das Sprachinteraktionssystem einen Lautsprecher in den Untersuchungsraum, eine Anzeigevorrichtung für einen Avatar des durch es realisierten virtuellen Gesprächspartners sowie Patientensensoren, umfassend optische Sensoren, insbesondere wenigstens eine Kamera und Abstandsmesssensoren. Die Steuereinrichtung des Sprachinteraktionssystems ist im hier beschriebenen Ausführungsbeispiel in eine Steuereinrichtung für die gesamte Bildaufnahmeeinrichtung integriert. Innerhalb der Steuereinrichtung weist das Sprachinteraktionssystem ferner eine Spracherkennungseinheit und eine Analyseeinheit auf. In der Spracherkennungseinheit wird ein Spracherkennungsalgorithmus eingesetzt, bei dem bereits künstliche Intelligenz eingesetzt werden kann. In jedem Fall ist in der Analyseeinheit ein Algorithmus der künstlichen Intelligenz realisiert, so dass auch Dialoge mit Patienten, beispielsweise spielerische Dialoge mit Kindern, geführt werden können.

In einem Schritt S1 des hier dargestellten Ausführungsbeispiels wird das Sprachinteraktionssystem auf einen aktuell zu untersuchenden Patienten kalibriert, so dass der Spracherkennungsalgorithmus Worte aus dessen Geräuschen ableiten kann. Diese Kalibrierung auf den aktuellen Patienten erfolgt während der Positionierung des Patienten auf einem Patientenpositionierungsmittel, insbesondere einer Patientenliege, mit Unterstützung einer Bedienperson, beispielsweise eines medizinisch-technischen Radiologieassistenten (MTRA). Eingeflochten in eine Unterhaltung mit dem Patienten sind dabei vorbestimmte Worte, wobei die Bedienperson den Patienten auch explizit bitten kann, die vorbestimmten Worte umfassende Initialisierungssätze zu sprechen. Mittels eines Bedienelements, das beispielsweise in einer Benutzeroberfläche eines von der Bedienperson für den Workflow ohnehin genutzten Tablets oder einer sonstigen Eingabevorrichtung realisiert sein kann, kann die Bedienperson der Spracherkennungseinheit ankündigen, dass nun die vorbestimmten Worte gesprochen werden. Diese werden mittels des Mikrofons aufgenommen und auf grundsätzlich bekannte Art und Weise zur Initialisierung auf den jeweiligen Patienten, mithin zur Kalibrierung, genutzt. Nach Abschluss des Schrittes S1 ist die Spracherkennungseinheit also in der Lage, Sprache des Patienten zu extrahieren und Worte zu erkennen.

Es sei noch angemerkt, dass im Schritt S1 zur Kalibrierung auch ein Mikrofon der Eingabevorrichtung, insbesondere des Tablets, als zusätzliche Datenquelle für die Aufnahme der vorbestimmten Worte benutzt werden kann.

In einem Schritt S2 werden, insbesondere dann, wenn die Bedienperson den Untersuchungsraum verlassen hat, Geräusche des Patienten mittels der Mikrofone aufgenommen. Gleichzeitig werden jedoch auch weitere Sensordaten gesammelt, die von den Patientensensoren aufgenommen werden. Die Analyse der Geräusche mittels der Spracherkennungseinheit sowie gegebenenfalls zusätzlich der Sensordaten erfolgt in einem Schritt S3.

Hierbei wird als Patienteninformation als Ergebnis der Auswertung der Geräusche eine Anfrageklasse ermittelt, der jeweils eine eine Sprachausgabe definierende Ausgabeinformation zugeordnet ist oder zugeordnet werden kann. Dabei werden verschiedenste Anfrageklassen im Rahmen dieses Ausführungsbeispiels eingesetzt. Insbesondere ist eine Rückfall-Anfrageklasse vorgesehen, falls die Geräusche bzw. darin enthaltenen Sprache nicht interpretiert werden können, mithin nicht mit hinreichender Sicherheit einer Anfrageklasse zugeordnet werden können. Dann kann beispielsweise eine entsprechende Sprachausgabe des Sprachinteraktionssystems in einem Schritt S4 erfolgen, die den Patienten bittet, eine nicht verstandene Sprache zu wiederholen, und/oder, insbesondere bei nichtinterpretierbaren Geräuschen, nachfragt, ob geholfen werden kann.

Eine weitere im Schritt S3 verwendete Anfrageklasse betrifft wenigstens eine beantwortbare Patientenfrage des Patienten. Insbesondere werden dabei als Patientenfragen entsprechender Anfrageklassen Anfragen zu einem Untersuchungsablauf verwendet. Beispielsweise kann ein Patient fragen, wann eine Untersuchung beginnt, wie lange die Untersuchung noch dauert, welcher Untersuchungsschritt gerade durchgeführt wird bzw. welcher Untersuchungsschnitt noch folgt und dergleichen. Auch andere Fragen können sich auf den Untersuchungsablauf beziehen, beispielsweise eine Anfrage des Patienten, ob er sich wieder bewegen darf und dergleichen. Zum Untersuchungsablauf, aber auch zu anderen Patientenfragen, können passende Antwortinformationen bereits in der Steuereinrichtung der Bildaufnahmeeinrichtung verfügbar sein. Daher ist es dem Sprachinteraktionssystem möglich, eine Ausgabeinformation, die die entsprechende Antwortinformation enthält, zu bilden, so dass mithin Patientenfragen auch ohne Anwesenheit einer Bedienperson automatisiert im S4 durch entsprechende Sprachausgabe beantwortet werden können. Beispielsweise kann dem Patienten eine noch verbleibende Untersuchungsdauer gemäß einem in der Steuereinrichtung abgelegten Untersuchungsprotokoll mitgeteilt werden.

Kann die Patientenfrage gemäß der Analyse im Schritt S3 nicht aufgrund einer in der Steuereinrichtung der Bildaufnahmeeinrichtung verfügbaren Antwortinformation beantwortet werden oder richtet sie sich explizit auf einen Kontaktwunsch mit einer menschlichen Bedienperson, wird zusätzlich zu einer Wartehinweis enthaltenen Sprachausgabe im Schritt S4 im Schritt S5 eine Maßnahme zur Kontaktierung wenigstens einer Bedienperson ergriffen. Hierzu wird vorliegend eine Anwesenheitsanfrage an eine Kommunikationseinrichtung der Bedienperson, im Beispiel ein Mobiltelefon und/oder ein DECT-Telefon, gesendet, um diese zu informieren, dass sie im Untersuchungsraum vom Patienten benötigt wird.

Auch unabhängig von konkreten, aktiven Fragen, also der Befriedigung eines konkreten Informationsbedürfnisses des Patienten, kann ein Unwohlsein des Patienten als implizite Anfrage verstanden werden und aus den Sensordaten der Patientensensoren sowie aus der Auswertung der Geräusche in der Spracherkennungseinheit folgen. Mithin wird als weitere Anfrageklasse wenigstens eine ein Unwohlsein des Patienten beschreibende Anfrageklasse verwendet. Hierbei sind zweckmäßiger Weise mehrere entsprechende, ein Unwohlsein des Patienten beschreibende Anfrageklassen vorgesehen, die sich auf verschiedene Arten und Grade des Unwohlseins beziehen können. Den entsprechenden, auf ein Unwohlsein bezogenen Anfrageklassen können dann unterschiedliche Maßnahmen zugeordnet sein. Während es allgemein sinnvoll ist, eine insbesondere auf die Art des Unwohlseins abgestimmte Sprachausgabe mit einer wohlwollenden, beruhigenden Stimme zu erzeugen, ist es beispielsweise bei Klaustrophobie des Patienten zweckmäßig, im Schritt S5 eine Notfallnachricht an die der wenigstens einen Bedienperson zugeordnete Kommunikationseinrichtung zu senden, insbesondere einen entsprechenden stillen Alarm. Bei anderen, kritischeren Arten des Unwohlseins, beispielweise einer erkannten Kontrastmittelunverträglichkeit, kann die entsprechende Notfallnachricht zusätzlich und/oder alternativ an einen Arzt, mithin eine diesem zugeordnete Kommunikationseinrichtung, gesendet werden, wiederum bevorzugt als stiller Alarm.

Es sei an dieser Stelle angemerkt, dass sich eine Einordnung in entsprechende Anfrageklassen auch allein aus einer Auswertung der Sensordaten der Patientensensoren, insbesondere also von Bilddaten und/oder Abstandsdaten, ergeben kann.

Als weitere Analyse wird im Schritt S3 im Übrigen auch überwacht, ob ein für den aktuellen Untersuchungsschritt, insbesondere Aufnahmeschritt, notwendiger Zielzustands des Patienten, beispielsweise eine bestimmte Positionierung, die zu Beginn der Untersuchung hergestellt wurde, und/oder ein Atemanhalten, beibehalten wird. Hierzu werden hauptsächlich die Sensordaten der Patientensensoren herangezogen. Wird ein drohendes oder stattfindendes Verlassen des Zielzustands des Patienten festgestellt, kann der Patient durch eine entsprechende Sprachausgabe im Schritt S4 angeregt werden, den Zielzustand dennoch beizubehalten oder wieder einzunehmen, während als sonstige Maßnahme im Schritt S5 beispielsweise die Bildaufnahme unterbrochen werden kann, bis der Patient des Zielzustand wieder eingenommen hat, worauf die Bildaufnahme neu beginnen kann bzw. fortgesetzt werden kann. Auch die Information einer Bedienperson ist wiederum möglich.

Parallel zu jeglicher Sprachausgaben im Schritt S4 wird eine entsprechende Animation eines grundsätzlich auf einer Anzeigevorrichtung angezeigten Avatars des virtuellen Gesprächspartners ausgespielt, wobei der Avatar beispielsweise auf eine Innenverkleidung einer Gantry und/oder Patientenaufnahme projiziert werden kann.

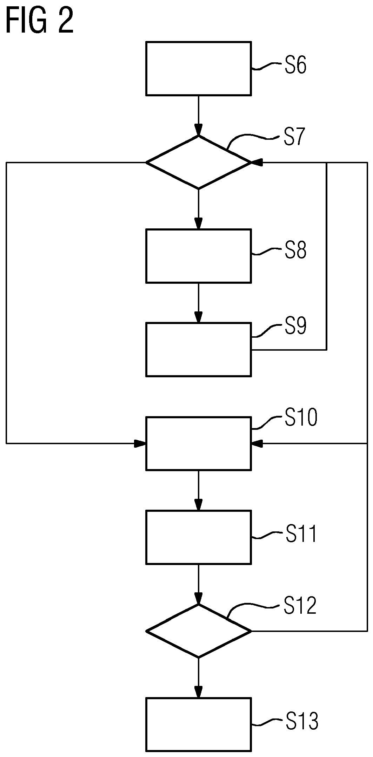

Es ist im Rahmen des hier beschriebenen Ausführungsbeispiels auch möglich, insbesondere bei pädiatrischen Patienten, einen gesamten, spielerischen Dialog mit dem Patienten, in diesem Fall ein Kind, zu führen, um dieses zu beschäftigen und/oder spielerisch anzuregen, eine Positionierung beizubehalten und/oder den Atem in Atemanhaltephasen der Untersuchung anzuhalten. Insbesondere kann in einem solchen Fall als Teil des Dialogs, aber auch im Allgemeinen, durch das Sprachinteraktionssystem eine Art "Coaching" hinsichtlich des Stillhaltens bzw. Atemanhaltens erfolgen. Dies wird durch Fig. 2 näher erläutert.
Wird in einem Schritt S6 dort festgestellt, dass die Untersuchung eine spezielle Atemanhaltephase und/oder Stillhaltephase umfasst, wird in einem Schritt S7 überprüft, ob schon ein hinreichendes Coaching vorliegt und/oder ob sich aus Patientendaten ergibt, dass ein entsprechendes Coaching nicht erforderlich ist. Handelt es sich bei dem Patienten beispielsweise um ein Kind oder eine aus anderen Gründen vermutlich wenig kooperative Person, wird jedoch ein Coaching durchgeführt, wozu in einem Schritt S8 zunächst eine Sprachausgabe erfolgt, die spielerisch sein kann. Beispielsweise kann der virtuelle Gesprächspartner eine Aussage tätigen wie "Ich wette, Du schaffst es nicht, zehn Sekunden den Atem anzuhalten."

In einem Schritt S9 werden wiederum Daten der Patientensensoren genutzt, um zu überprüfen, ob sich der entsprechend angefragte Zielzustand (Stillhalten / Atem anhalten / ...) ergibt. Ist dies für eine vorbestimmte Zeitspanne der Fall, kann das Coaching als erfolgreich bewertet werden, ansonsten als gescheitert. Im nun wiederum folgenden Schritt S7 können verschiedene Kriterien definiert und überprüft werden, ob das Coaching erfolgreich war, beispielsweise eine bestimmte Zahl an erfolgreichen Beibehalten des Zielzustandes für die vorbestimmte Zeitspanne und dergleichen.
Sobald ein entsprechendes Kriterium erfüllt ist, wird im Schritt S10 fortgefahren, wo wiederum eine Sprachausgabe des virtuellen Gesprächspartners erfolgt, mit der gegebenenfalls spielerisch formulierten Bitte, den Zielzustand einzunehmen und für die vorbestimmte Zeitspanne beizubehalten. In einem Schritt S11 werden wiederum entsprechende Sensordaten mit den Sensoren aufgenommen, anhand derer überprüft werden kann, ob der Zielzustand tatsächlich eingenommen ist. Wird im Schritt S12 festgestellt, dass dies nicht der Fall ist, kann entweder wieder zum Coaching in Schritt S7 zurückverzweigt werden, oder aber die Aufforderung entsprechend wiederholt werden, mithin zu Schritt S10 zurückverzweigt werden. Dabei kann dem Patienten ein gewisses Zeitintervall zugestanden werden, den Zielzustand einzunehmen und bereits zu halten. Ist der Zielzustand eingenommen, wird im Schritt S13 ein Triggersignal zur entsprechenden tatsächlichen Bildaufnahme innerhalb der Steuereinrichtung der Bildaufnahmeeinrichtung erzeugt, welches an eine entsprechende Bildaufnahmeeinheit gesendet werden kann.

Wie im Hinblick auf Fig. 1 erläutert wurde, kann selbstverständlich auch bei der im Schritt S13 erfolgenden Bildaufnahme weiterhin überwacht werden, ob der Zielzustand beibehalten wird, wobei bei Verlassen des Zielzustandes wieder entsprechende Maßnahmen ergriffen werden können.

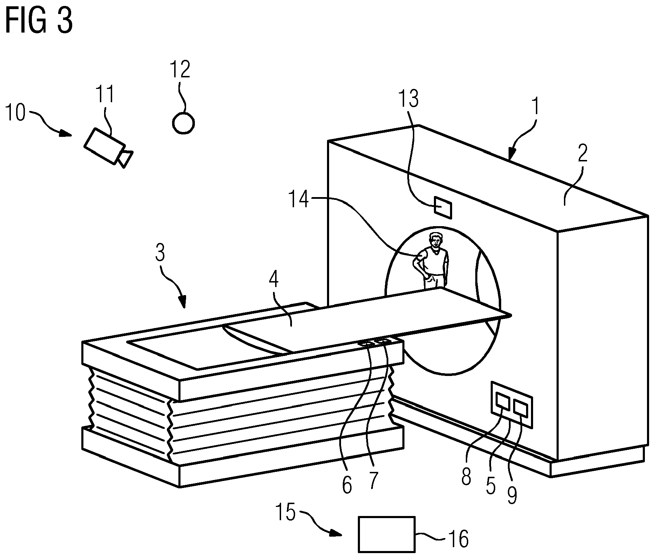

Fig. 3 zeigt schließlich eine Prinzipskizze einer erfindungsgemäßen medizinischen Bildaufnahmeeinrichtung 1, vorliegend einer Computertomographieeinrichtung. Diese weist, wie grundsätzlich bekannt, eine Gantry 2 auf, in die ein Patient mittels eines Patientenpositionierungsmittels 3, hier einer Patientenliege 4, eingefahren werden kann. Der Betrieb der Bildaufnahmeeinrichtung wird mittels einer Steuereinrichtung 5 gesteuert, die vorliegend auch als Steuereinrichtung des Sprachinteraktionssystems dient. Dieses umfasst neben den hier beispielhaft an der Patientenliege 4 dargestellten Komponenten des Mikrofons 6 und des Lautsprechers 7 als Teil der Steuereinrichtung 5 die Spracherkennungseinheit 8 und die Analyseeinheit 9. Ferner sind dem Sprachinteraktionssystem auch Patientensensoren 10 zugeordnet, vorliegend eine Kamera 11 als optischer Patientensensor 10 und wenigstens ein Abstandsensor 12. Es versteht sich, dass weitere Patientensensoren 10, Mikrofone 6 und/oder Lautsprecher 7 eingesetzt werden können.

Das Sprachinteraktionssystem weist ferner eine Anzeigevorrichtung 13 auf, vorliegend eine Projektionseinheit, über die ein Avatar 14 des virtuellen Gesprächspartners auf eine Innenverkleidung der Gantry 2 projiziert werden kann. Auch die Verwendung einer Holographieeinrichtung ist denkbar, wobei der Avatar 14 dann als Hologramm ausgebildet ist.

Alternativ kann es sich bei der Anzeigevorrichtung 13 auch um eine Augmented-Reality-Brille oder eine Virtual-Reality-Brille handeln, die bevorzugt den Avatar 14 in eine virtuelle Alternativumgebung statt dem Untersuchungsraum projiziert, beispielsweise eine Waldlandschaft. Lokalisierte Objekte des Untersuchungsraums können zur Vermeidung von Kollisionen als zu umgehende Objekte der Alternativumgebung überblendet werden, beispielsweise als Bäume, Interaktionsobjekte, wie beispielsweise die Patientenliege 4, als entsprechend (insbesondere mittels des Avatars 14) zur Interaktion einladende virtuelle Objekte, beispielsweise als gemütliches Bett im Fall der Patientenliege 4.

Der Bildaufnahmeeinrichtung 1 zugeordnet ist ferner als Eingabevorrichtung 15 für eine Bedienperson ein Tablet-PC 16, auf dem auch insgesamt ein Softwaremittel zum Workflow-Management vorgesehen sein kann.

Die Steuereinrichtung 5 ist insgesamt zur Durchführung des bezüglich der Fig. 1 und 2 beschriebenen Ausführungsbeispiels des erfindungsgemäßen Verfahrens ausgebildet.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Betrieb einer medizinischen Bildaufnahmeeinrichtung (1) im Rahmen einer Untersuchung eines mit einem Patientenpositionierungsmittel (3) positionierten Patienten, **dadurch gekennzeichnet, dass** die Bildaufnahmeeinrichtung (1) ein Sprachinteraktionssystem mit wenigstens einem Geräusche des Patienten aufnehmenden Mikrofon (6) und wenigstens einem Lautsprecher (7) zu dem Patienten aufweist, wobei
- von dem Patienten stammende Geräusche mittels des Mikrofons (6) aufgenommen werden und von einer Spracherkennungseinheit (8) des Sprachinteraktionssystems zur Ermittlung einer den Zustand des Patienten und/oder den Inhalt gesprochener Worte beschreibenden Patienteninformation ausgewertet werden,
- in Abhängigkeit von der Patienteninformation eine Sprachausgabe über den Lautsprecher (7) an den Patienten erfolgt.

2. Verfahren nach Anspruch 1, **dadurch gekennzeichnet, dass** bei der Auswertung der Geräusche als Patienteninformation eine Anfrageklasse ermittelt wird, wobei den Anfrageklassen eine die Sprachausgabe definierende Ausgabeinformation zugeordnet ist oder wird.

3. Verfahren nach Anspruch 2, **dadurch gekennzeichnet, dass** wenigstens eine eine beantwortbare Patientenfrage des Patienten beschreibende Anfrageklasse verwendet wird.

4. Verfahren nach Anspruch 3, **dadurch gekennzeichnet, dass** bei einer eine in einer Steuereinrichtung (5) der Bildaufnahmeeinrichtung (1) vorliegenden, der Patientenfrage zuordenbaren Antwortinformation die Ausgabeinformation unter Aufnahme der Antwortinformation gebildet wird.

5. Verfahren nach Anspruch 3 oder 4, **dadurch gekennzeichnet, dass** bei einer einen Kontaktwunsch mit einem menschlichen Bedienpersonal ausdrückenden Patientenfrage eine Anwesenheitsanfrage an eine Kommunikationseinrichtung wenigstens einer Bedienperson gesendet und ein, insbesondere den Namen der kontaktierten Bedienperson enthaltender, Wartehinweis als Sprachausgabe an den Patienten ausgegeben wird.

6. Verfahren nach einem der Ansprüche 2 bis 5, **dadurch gekennzeichnet, dass** wenigstens eine ein Unwohlsein des Patienten beschreibende Anfrageklasse verwendet wird, wobei als Ausgabeinformation ein den Patienten beruhigender Ansprachetext mit wohlwollender Stimmlage ausgegeben wird und eine Notfallnachricht an wenigstens eine wenigstens einer Bedienperson zugeordnete Kommunikationseinrichtung ausgegeben wird.

7. Verfahren nach einem der vorangehenden Ansprüche, **dadurch gekennzeichnet, dass**, insbesondere bei einem Kind als Patienten, ein den Patienten beschäftigender, spielerischer Dialog geführt wird, insbesondere durch einen Algorithmus der künstlichen Intelligenz.

8. Verfahren nach einem der vorangehenden Ansprüche, **dadurch gekennzeichnet, dass** wenigstens eine Sprachausgabe, insbesondere ein Dialogabschnitt des Dialogs, dem Herstellen eines Zielzustandes des Patienten in Abhängigkeit von einem Untersuchungsprotokoll dient.

9. Verfahren nach Anspruch 8, **dadurch gekennzeichnet, dass** der Zielzustand ein Atemanhalten und/oder ein Stillhalten für eine vorbestimmte Zeitspanne betrifft, wobei das Herstellen des Zielzustandes als ein Triggersignal für eine die Untersuchung steuernde Steuereinrichtung (5) der Bildaufnahmeeinrichtung (1) verwendet wird.

10. Verfahren nach Anspruch 8 oder 9, **dadurch gekennzeichnet, dass** die Herstellung des Zielzustandes durch die Auswertung von Sensordaten wenigstens eines den Patienten überwachenden Patientensensors (10) überprüft wird.

11. Verfahren nach einem der vorangehenden Ansprüche, **dadurch gekennzeichnet, dass** zur Kalibrierung der Spracherkennungseinheit (8) auf einen Patienten vorbestimmte, von dem Patienten gesprochene Worte aufgenommen und zur Kalibrierung analysiert werden.

12. Verfahren nach Anspruch 11, **dadurch gekennzeichnet, dass** bei Einflechtung der vorbestimmten Worte in einen Dialog des Patienten mit einer Bedienperson und/oder Anforderung der vorbestimmten Worte durch die Bedienperson mittels einer Eingabevorrichtung ein das Sprechen der vorbestimmten Worte anzeigendes Kalibrierungssignal nach einer entsprechenden Betätigung eines Bedienelements durch die Bedienperson erzeugt und an die Spracherkennungseinheit (8) geschickt wird.

13. Verfahren nach Anspruch 12, **dadurch gekennzeichnet, dass** die Eingabevorrichtung selbst wenigstens ein Mikrofon (6) aufweist, das zusätzlich zu dem Mikrofon (6) des Sprachinteraktionssystems die vorbestimmten Worte aufnimmt, wobei die jeweiligen Aufnahmen gemeinsam zur Kalibrierung ausgewertet werden.

14. Verfahren nach einem der vorangehenden Ansprüche, **dadurch gekennzeichnet, dass** bei der Auswertung der Geräusche zusätzlich Bilddaten eines auf den Patienten gerichteten optischen Patientensensors (10) berücksichtigt werden, insbesondere bei Verwendung einer ein Unwohlsein des Patienten beschreibenden Anfrageklasse.

15. Verfahren nach einem der vorangehenden Ansprüche, **dadurch gekennzeichnet, dass** eine Anzeigevorrichtung (13) zur Anzeige eines mit Sprachausgaben über den Lautsprecher (7) synchronisierten Avatars (14) des Sprachinteraktionssystems verwendet wird.

16. Verfahren nach Anspruch 15, **dadurch gekennzeichnet, dass** als Anzeigevorrichtung (13) eine Augmented-Reality-Brille oder eine Virtual-Reality-Brille verwendet wird, die der Patient trägt.

17. Verfahren nach Anspruch 16, **dadurch gekennzeichnet, dass** die Augmented-Reality-Brille oder Virtual-Reality-Brille zur Darstellung einer beruhigend auf den Patienten wirkenden Alternativumgebung verwendet wird, wobei insbesondere in einem Untersuchungsraum, in dem die Untersuchung stattfindet, vorhandene Gegenstände, insbesondere durch eine brillenseitige Sensorik, detektiert und durch Objekte der Alternativumgebung ersetzt werden.

18. Verfahren nach einem der Ansprüche 15 bis 17, **dadurch gekennzeichnet, dass** der Avatar (14) auf das Innere einer Patientenaufnahme und/oder Gantry (2) projiziert wird und/oder auf wenigstens einem Display als Anzeigevorrichtung (13) dargestellt wird und/oder dass die Anzeigevorrichtung (13) als Holograpgieeinrichtung ausgebildet ist, die den Avatar (14) als Hologramm anzeigt.

19. Bildaufnahmeeinrichtung (1), aufweisend ein zur Durchführung eines Verfahrens nach einem der vorangehenden Ansprüche ausgebildetes Sprachinteraktionssystem.

20. Computerprogramm, welches die Schritte eines Verfahrens nach einem der Ansprüche 1 bis 18 durchführt, wenn es auf einer Recheneinrichtung ausgeführt wird.

21. Elektronisch lesbarer Datenträger, auf dem ein Computerprogramm nach Anspruch 20 gespeichert ist.

## Claims

1. Method for operating a medical image recording device (1) within the scope of an examination of a patient positioned with a patient poisoning means (3), **characterised in that** the image recording device (1) has a speech interaction system with at least one microphone (6) recording a noise of the patient and at least one loudspeaker (7) for the patient, wherein
- noise coming from the patient is recorded by means of the microphone (6) and is evaluated by a speech recognition unit (8) of the speech interaction system in order to determine an item of patient information describing the state of the patient and/or the content of spoken words,
- a speech output is carried out via the loudspeaker (7) to the patient as a function of the patient information.

2. Method according to claim 1, **characterised in that** with the evaluation of the noises as patient information, a query class is determined, wherein an item of output information defining the speech output is or will be assigned to the query classes.

3. Method according to claim 2, **characterised in that** at least one query class describing an answerable patient query of the patient is used.

4. Method according to claim 3, **characterised in that** with an item of response information present in a control device (5) of the image recording device (1) and assignable to the patient query, the output information is formed by recording the response information.

5. Method according to claim 3 or 4, **characterised in that** with a patient query expressing the desire for contact with a human operator, a presence query is sent to a communication device of at least one operator, and a wait message containing in particular the name of the contacted operator is output as speech output to the patient.

6. Method according to one of claims 2 to 5, **characterised in that** at least one query class describing an illness of the patient is used, wherein as output information a speech text calming the patient is output with a sympathetic pitch of the voice and an emergency message is output to at least one communication device assigned to at least one operator.

7. Method according to one of the preceding claims, **characterised in that** particularly with a child as a patient, a playful dialog occupying the patient is conducted, in particular by an artificial intelligence algorithm.

8. Method according to one of the preceding claims, **characterised in that** at least one speech output, in particular a dialog section of the dialog, is used to produce a target state of the patient as a function of an examination protocol.

9. Method according to claim 8, **characterised in that** the target state relates to breath holding and/or keeping still for a predetermined period of time, wherein the production of the target state is used as a trigger signal for a control device (5) of the image recording device (1) which controls the examination.

10. Method according to claim 8 or 9, **characterised in that** the production of the target state is checked by evaluating sensor data of at least one patient sensor (10) monitoring the patient.

11. Method according to one of the preceding claims, **characterised in that** in order to calibrate the voice recognition unit (8) to a patient, predetermined words spoken by the patient are recorded and analysed for calibration purposes.

12. Method according to claim 11, **characterised in that** when the predetermined words are inserted into a dialog of the patient with an operator and/or request of the predetermined words by the operator by means of an input device, a calibration signal indicating the speech of the predetermined words is generated after a corresponding actuation of an operating element by the operator and is sent to the speech recognition unit (8).

13. Method according to claim 12, **characterised in that** the input device itself has at least one microphone (6), which, in addition to the microphone (6) of the speech interaction system, records the predetermined words, wherein the respective recordings are evaluated together for calibration purposes.

14. Method according to one of the preceding claims, **characterised in that** when the noises are evaluated, additional image data of an optical patient sensor (10) directed at the patient is taken into account, in particular when a query class describing an illness of the patient is used.

15. Method according to one of the preceding claims, **characterised in that** a display device (13) is used to display an avatar (14) of the speech interaction system which is synchronised with speech outputs by way of the loud speaker (7) .

16. Method according to claim 15, **characterised in that** augmented reality glasses or virtual reality glasses worn by the patient are used as a display device (13).

17. Method according to claim 16, **characterised in that** augmented reality glasses or virtual reality glasses are used to display an alternative environment acting in a calming manner on the patient, wherein in particular in an examination room, in which the examination takes place, available objects are detected in particular by a sensor system in the glasses and are replaced by objects in the alternative environment.

18. Method according to one of claims 15 to 17, **characterised in that** the avatar (14) is projected onto the interior of a patient receptacle and/or gantry (2) and/or is shown on at least one display as a display device (13) and/or that the display device (13) is embodied as a holography device which indicates the avatar (14) as a hologram.

19. Image recording device (1), having a speech interaction system embodied to carry out a method according to one of the preceding claims.

20. Computer program, which carries out the steps of a method according to one of claims 1 to 18, when it is executed on a computer device.

21. Electronically readable data carrier, on which a computer program according to claim 20 is stored.

## Revendications

1. Procédé pour faire fonctionner un dispositif (1) d'enregistrement d'image médicale dans le cadre d'un examen d'un patient mis en position par un moyen (3) de mise en position de patient, **caractérisé en ce que** le dispositif (1) d'enregistrement d'image a un système d'interaction vocale, ayant au moins un microphone (6) d'enregistrement de bruits du patient et au moins un haut parleur (7) pour le patient, dans lequel
- on enregistre, au moyen du microphone (6), des bruits provenant du patient et on les exploite par une unité (8) de reconnaissance vocale du système d'interaction vocale, afin de déterminer une information de patient décrivant l'état du patient et/ou le contenu des mots prononcés,
- une émission vocale vers le patient par l'intermédiaire du haut parleur (7) est effectuée en fonction de l'information de patient.

2. Procédé suivant la revendication 1, **caractérisé en ce que**, lors de l'exploitation des bruits, on détermine, comme information de patient, une classe de demande, une information d'émission définissant l'émission vocale étant associée aux classes de demande.

3. Procédé suivant la revendication 2, **caractérisé en ce que** l'on utilise au moins une classe de demande décrivant une demande du patient, à laquelle il peut être répondu.

4. Procédé suivant la revendication 3, **caractérisé en ce que**, s'il y a une information de réponse présente dans un dispositif (5) de commande du dispositif (1) d'enregistrement d'image et pouvant être associée à la demande du patient, on forme l'information d'émission en enregistrant l'information de réponse.

5. Procédé suivant la revendication 3 ou 4, **caractérisé en ce que**, s'il y a une demande de patient exprimant un souhait de contact avec un personnel humain de service, on envoie une demande de présence à un dispositif de communication d'au moins une personne de service et on émet, comme émission vocale vers le patient, une indication d'attente, contenant notamment le nom de la personne de service contactée.

6. Procédé suivant l'une des revendications 2 à 5, **caractérisé en ce que** l'on utilise au moins une classe de demande décrivant une indisposition du patient, dans lequel on émet, comme information d'émission, un texte parlé tranquillisant le patient, de ton bienveillant, et on émet un message d'urgence vers au moins un dispositif de communication associé à au moins une personne de service.

7. Procédé suivant l'une des revendications précédentes, **caractérisé en ce que**, notamment lorsque le patient est un enfant, on conduit un dialogue enjoué et occupant le patient, notamment par un algorithme de l'intelligence artificielle.

8. Procédé suivant l'une des revendications précédentes, **caractérisé en ce qu'**au moins une émission vocale, notamment une partie du dialogue, sert à préparer un état cible du patient en fonction d'un programme d'examen.

9. Procédé suivant la revendication 8, **caractérisé en ce que** l'état cible concerne un maintien de la respiration et/ou un repos pendant un laps de temps déterminé à l'avance, la production de l'état cible étant utilisée comme signal de déclenchement d'un dispositif (5) de commande du dispositif (1) d'enregistrement d'image commandant l'examen.

10. Procédé suivant la revendication 8 ou 9, **caractérisé en ce que** l'on contrôle la production de l'état cible par l'exploitation de données de capteur d'au moins un capteur (10) de patient contrôlant le patient.

11. Procédé suivant l'une des revendications précédentes, **caractérisé en ce que** pour étalonner l'unité (8) de reconnaissance vocale, on enregistre des mots déterminés à l'avance sur un patient et prononcés par le patient et on les analyse pour l'étalonnage.

12. Procédé suivant la revendication 11, **caractérisé en ce que**, lors de l'insertion des mots déterminés à l'avance dans un dialogue du patient avec une personne de service et/ou lors de la demande des mots déterminés à l'avance par la personne de service, on produit, après un actionnement adéquat d'un élément de commande par la personne de service, un signal d'étalonnage indiquant la prononciation des mots déterminés à l'avance et on l'envoie à l'unité (8) de reconnaissance vocale.

13. Procédé suivant la revendication 12, **caractérisé en ce que** le dispositif d'envoi a soi-même au moins un microphone (6), qui enregistre, en plus du microphone (6) du système d'interaction vocale, les mots déterminés à l'avance, les enregistrements respectifs étant exploités conjointement pour l'étalonnage.

14. Procédé suivant l'une des revendications précédentes, **caractérisé en ce que**, lors de l'exploitation des bruits, on prend en compte, supplémentairement, des données d'image d'un capteur (10) optique de patient dirigé sur le patient, notamment en utilisant une classe de demande décrivant une indisposition du patient.

15. Procédé suivant l'une des revendications précédentes, **caractérisé en ce que** l'on utilise un dispositif (13) d'affichage pour afficher un avatar (14), synchronisé avec des émissions vocales par l'intermédiaire du haut-parleur (7), du système d'interaction vocale.

16. Procédé suivant la revendication 15, **caractérisé en ce que** l'on utilise comme dispositif (13) d'affichage une lunette à réalité augmentée ou une lunette à réalité virtuelle que porte le patient.

17. Procédé suivant la revendication 16, **caractérisé en ce que** l'on utilise la lunette à réalité augmentée ou la lunette à réalité virtuelle pour représenter un environnement alternatif à effet tranquillisant sur le patient, dans lequel, notamment dans un espace d'examen dans lequel a lieu l'examen, on détecte des objets présents, notamment par un équipement de capteur du côté de la lunette, et on les remplace par des objets de l'environnement alternatif.

18. Procédé suivant l'une des revendications 15 à 17, **caractérisé en ce que** l'on projette l'avatar (14) sur l'intérieur d'un enregistrement de patient et/ou un portique (2) et/ou on le représente sur au moins un écran comme dispositif (13) d'affichage et/ou **en ce que** le dispositif (13) d'affichage est constitué sous la forme d'un dispositif holographique, qui indique l'avatar (14) sous la forme d'un hologramme.

19. Dispositif (1) d'enregistrement d'image ayant un système d'interaction vocale constitué pour effectuer un procédé suivant l'une des revendications précédentes.

20. Programme d'ordinateur, qui effectue les stades d'un procédé suivant l'une des revendications 1 à 18, lorsqu'il est exécuté sur un dispositif de calcul.

21. Support de données déchiffrable électroniquement, sur lequel est mis en mémoire un programme d'ordinateur suivant la revendication 20.
